**Europäisches Patentamt**

**European Patent Office**

**Office européen des brevets**

(19)

(11) Veröffentlichungsnummer: **0 035 799**
**B1**

(12)

# EUROPÄISCHE PATENTSCHRIFT

(45) Veröffentlichungstag der Patentschrift:
**11.07.84**

(21) Anmeldenummer: **81102638.4**

(22) Anmeldetag: **30.01.80**

(60) Veröffentlichungsnummer der früheren Anmeldung
nach Art. 76 EPÜ:

(51) Int. Cl.³: **C 07 C 99/02** // C07C101/08

(54) **Verfahren zur Gewinnung von Phenylalanin.**

(43) Veröffentlichungstag der Anmeldung:
**16.09.81 Patentblatt 81/37**

(45) Bekanntmachung des Hinweises auf die Patenterteilung:
**11.07.84 Patentblatt 84/28**

(84) Benannte Vertragsstaaten:
**BE FR GB NL**

(56) Entgegenhaltungen:
**DE - A - 1 127 358**
**FR - A - 937 909**
**US - A - 2 422 938**

(73) Patentinhaber: **SOCIETE DES PRODUITS NESTLE S.A.,**
**Case postale 353, CH-1800 Vevey (CH)**

(72) Erfinder: **Steinmetzer, Walter, Im Bodetal 9,**
**D-3334 Süpplingen (DE)**

## Beschreibung

Die vorliegende Erfindung betrifft ein Verfahren zur Gewinnung von Phenylalanin aus Proteinhydrolysaten, bei dem Aminosäurehydrochloride aus wässrigen Lösungen mit einem mit Wasser nicht mischbaren Alkohol, insbesondere Isobutanol oder Butanol extrahiert werden.

Ziel der vorliegenden Erfindung ist eine industriell verwertbare Gewinnung von L-Phenylalanin sehr hoher Reinheit für die Verwendung in der parenteralen Ernährung.

Zu diesem Zweck ist das erfindungsgemässe Verfahren dadurch gekennzeichnet, dass ein Proteinhydrolysat bei einem pH-Wert von 0,5 bis 1,0 mit Kochsalz gesättigt, mit dem genannten Alkohol Leucin-, Isoleucin- und Phenylalaninhydrochlorid neben geringem Anteil an Tyrosin- und Valinhydrochlorid extrahiert, durch Abdestillation des Alkohols eine Lösung der genannten Aminosäurehydrochloride erhalten, diese Lösung über einen schwach basischen und über einen stark basischen Anionenaustauscher geleitet, der mit Phenylalanin und Tyrosin beladene stark basische Anionenaustauscher mit verdünnter Salzsäure eluiert, das phenylalanin- und tyrosinhaltige Eluat konzentriert, dessen Salzsäuregehalt auf 20-25% eingestellt, und nach Abtrennung von Natriumchlorid bei 70 bis 90°C Phenylalaninhydrochlorid durch Kühlung auskristallisiert und durch Umkristallisation aus 20 bis 25%iger Salzsäure weiter gereinigt wird. Dann kann das reine Phenylalaninhydrochlorid in Wasser gelöst, mit einem schwach basischen Anionenaustauscher die Salzsäure abgetrennt und durch Konzentrieren der neutralen Lösung reines L-Phenylalanin gewonnen werden. Vorzugsweise wird noch durch Waschen des Alkoholextraktes mit Wasser dessen Gehalt an Tyrosin und Valin vermindert.

Es wurde nämlich gefunden, dass die Hydrochloride von Phenylalanin, Leucin und Isoleucin aus kochsalzhaltigen Lösungen mit einem mit Wasser nicht mischbaren Alkohol, insbesondere Isobutanol oder Butanol, mit sehr guten Ausbeuten extrahierbar sind. Die Extraktion der Aminosäurehydrochloride aus wässrigen Lösungen mit Butanol ist bekannt. Diese Extraktion ergibt jedoch geringe Ausbeuten, da die Verteilungskoeffizienten der Aminosäurehydrochloride im System Butanol/Wasser unter 1,0 liegen. Da bei diesen Extraktionsausbeuten das Verfahren unwirtschaftlich ist, wurde der Einfluss verschiedener Zusätze auf die Extraktionsausbeuten überprüft.

Mit dem in der DE-B-1 127 358 beschriebenen begrenzten Zusatz verschiedener anorganischer Salze konnten nur geringfügig Verbesserungen erzielt werden. Es wurde nun gefunden, dass erst bei Sättigung der Lösung mit Kochsalz sich die Verteilungskoeffizienten für Phenylalanin, Leucin und Isoleucin von 1,0 auf 10,0 erhöhen.

Als Ausgangsmaterial für die Durchführung des vorliegenden Verfahrens kann jedes beliebige Proteinhydrolysat verwendet werden. Diese Proteinhydrolysate werden durch salzsaure Hydrolyse verschiedener Rohstoffe wie Getreidekleber, Sojaschrot, Erdnussschrot, Öltrester, Hefe oder Kasein erhalten, und für die erfindungsgemässe Extraktion wird ein pH-Wert von 0,5 bis 1,0 durch Neutralisation mit Natronlauge oder Natriumkarbonat eingestellt und diese Lösung mit Kochsalz gesättigt. Bevorzugtes Ausgangsmaterial sind die Mutterlaugen der in der europäischen Patentanmeldung Nr. 80100459.9 beschriebenen Leucinfällung, welche Mutterlaugen die Aminosäuren Isoleucin, Phenylalanin, Tyrosin, Valin und restliche Anteile an Leucin enthalten.

So wird hier die mit Kochsalz gesättigte, einen pH-Wert von 0,5 bis 1,0 aufweisende Ausgangslösung mit einem mit Wasser nicht mischbaren Alkohol, insbesondere Isobutanol oder Butanol extrahiert. Bei dieser erfindungsgemässen Durchführung der Extraktion können 90-95% des Leucins, Isoleucins und Phenylalanins, 40-45% des Tyrosins und Valins und die möglichen restlichen Aminosäuren in kaum messbarem Umfang extrahiert werden. Durch Waschen des Alkoholextraktes mit Wasser kann der Anteil an Valin und Tyrosin weiter reduziert werden. Dieses Waschen mit Wasser kann direkt am Kopf der Extraktionskolonne oder in einer gesonderten Apparatur erfolgen. Aus dem Alkoholextrakt kann nach Zusatz von Wasser der Alkohol azeotrop abdestilliert und in die Extraktionskolonne zurückgeführt werden. Die wässrige Lösung enthält Aminosäurehydrochloride von Leucin, Isoleucin und Phenylalanin mit geringen Anteilen an Tyrosin und Valin.

Nach dem üblichen Stand der Technik wird Phenylalanin gemeinsam mit Tyrosin durch Adsorption an Aktivkohle aus sauren Lösungen gewonnen. Dieses Verfahren hat verschiedene Nachteile: so muss einmal die Aktivkohle durch Behandlung mit Essigsäure aktiviert werden, die adsorbierte Menge an Phenylalanin, bezogen auf das Aktivkohlevolumen, ist sehr gering, und die Eluierung des Phenylalanins erfolgt mit organischen Lösungsmitteln wie Pyridin oder Anilin.

Es ist bekannt, dass Aminosäuren von Kationen- oder Anionenaustauschern mit unterschiedlicher Stärke adsorbiert werden. Bei der systematischen Überprüfung der verschiedenen Ionenaustauschertypen wurde gefunden, dass die aromatischen Aminosäuren Phenylalanin und Tyrosin durch stark basische Anionenaustauscher vom Gel-Typ besonders stark adsorbiert werden. Diese starke selektive Adsorption konnte weder bei Kationenaustauschern, schwach basischen Anionenaustauschern und stark basischen makroporösen Ionenaustauschern beobachtet werden.

Durch diese nicht vorhersehbare selektive Adsorption der aromatischen Aminosäuren an einem stark basischen Anionenaustauscher in der OH-Form wurde eine wesentliche verbesserte Methode zur Gewinnung von Phenylalanin gefunden. Im Verhältnis zu der bekannten Kohleadsorption kann hierbei die fünffache Menge an Phenylalanin pro Volumeneinheit aufgenommen werden.

So wird hier die genannte Lösung der Hydrochloride der betreffenden Aminosäuren über einen schwach basischen und über einen stark basischen Anionenaustauscher geleitet.

Es wurde weiterhin gefunden, dass durch einfaches Eluieren des stark basischen Anionenaustauschers mit einer Salzsäurelösung das Phenylalanin und das Tyrosin vollständig vom Ionenaustauscher entfernt werden können. Bei der üblichen Regeneration mit einer 4%igen Natronlauge wird das Phenylalanin und Tyrosin nur teilweise entfernt.

Die Eluate von Anionenaustauscher enthalten die Hydrochloride des Phenylalanins und Tyrosins. Bei der Überprüfung des Löslichkeitsverhaltens in 6 n HCl wurde gefunden, dass Phenylalaninhydrochlorid in der Kälte schwer löslich und in der Wärme gut löslich ist. Tyrosin ist in der Kälte in 6 n HCl leichter löslich. So wird hier das Eluat konzentriert, dessen Salzsäuregehalt auf 20-25% eingestellt und nach Abtrennung von Natriumchlorid bei 70 bis 90°C Phenylalaninhydrochlorid durch Kühlung auskristallisiert und durch Umkristallisation aus 20 bis 25%iger Salzsäure weiter gereinigt.

Nach dem Eindampfen des salzsauren Eluats vom stark basischen Anionenaustauscher kann in der ersten Kristallisationsstufe eine Ausbeute von 85% und eine Reinheit von 90-92% erreicht werden. Durch Umkristallisation aus 20 bis 25%iger Salzsäure kann in der zweiten Kristallisationsstufe eine Ausbeute von 90% und eine Reinheit von 99% erreicht werden. Das reine Phenylalaninhydrochlorid kann dann in Wasser gelöst und zur Abtrennung der Salzsäure über einen schwach basischen Austauscher geleitet werden.

*Beispiel*

Man geht von einer Aminosäurelösung aus, welche die folgende in relativen Gewichtsprozenten ausgedrückte Zusammensetzung aufweist: Phe 15,9; Leu 19,2; Ile 16,0; Val 8,0; Tyr 6,5; Met 7,7; Asp 3,8; Thr 1,4; Ser 2,5; Glu 9,1; Pro 2,5; Gly 1,8 und Ala 5,6. Diese Ausgangslösung wird mit Salzsäure auf einen pH-Wert von 0,5 bis 1,0 eingestellt, mit Kochsalz gesättigt und in einer Extraktionskolonne im Gegenstrom mit Isobutanol extrahiert. Das Verhältnis Isobutanol zur Ausgangslösung wird auf 1:1 oder 1:2 eingestellt. Der Isobutanolextrakt weist die folgende Zusammensetzung auf, wobei die Phenylalanin-Ausbeute 95% ausmacht: Phe 28,0; Leu 32,9; Ile 26,8; Val 5,9; Tyr 5,4; andere zusammmen 1,0.

Durch Waschen des Isobutanolextraktes mit Wasser wird der Anteil an Valin und Tyrosin weiter reduziert. Dieses Waschen mit Wasser erfolgt direkt auf dem Kopf der Extraktionskolonne. Aus dem Isobutanolextrakt wird nach Zusatz von Wasser das Isobutanol azeotrop abdestilliert und in die Extraktionskolonne zurückgeführt. Aus der wässrigen Lösung der Aminosäurehydrochloride wird mit einem schwach basischen Anionenaustauscher die Salzsäure entfernt und die neutrale Aminosäurelösung wird über einen stark basischen Anionenaustauscher in der Hydroxydform geleitet. Im Auslauf der Säule wird eine reine Lösung von Leucin, Isoleucin und Valin erhalten. Die mit Phenylalanin und Tyrosin beladene Anionenaustauschersäule wird mit 1 Bettvolumen einer 10%igen Salzsäure und anschliessend mit 1 Bettvolumen 4%iger Natronlauge regeneriert. Die Eluate von der Salzsäureregeneration werden gesammelt und konzentriert. Durch Zusatz konzentrierter Salzsäure wird die Säurekonzentration auf mindestens 20% eingestellt und das vorhandene Kochsalz bei einer Temperatur von 70 bis 90°C abgetrennt. Nach dem Abkühlen der Lösung kristallisiert Phenylalaninhydrochlorid aus. Letzteres enthält die verschiedenen Aminosäurehydrochloride im folgenden Verhältnis bei einer auf den Isobutanolextrakt bezogenen Phenylalanin-Ausbeute von 85%: Phe 95,1; Leu 1,3; Ile 0,9; Val 0,0 und Tyr 2,6. Es wird durch Umkristallisation aus 20%iger Salzsäure weiter gereinigt und man erreicht in dieser zweiten Kristallisation eine Reinheit von 99% bei einer Ausbeute von 90%.

Das reine Phenylalanin wird dann in Wasser gelöst und zur Abtrennung der Salzsäure über einen schwach basischen Austauscher geleitet. Beim Konzentrieren der neutralen Lösung kristallisiert reines L-Phenylalanin aus.

**Patentansprüche**

1. Verfahren zur Gewinnung von L-Phenylalanin aus Proteinhydrolysaten, bei dem Aminosäurehydrochloride aus wässrigen Lösungen mit einem mit Wasser nicht mischbaren Alkohol, insbesondere Isobutanol oder Butanol extrahiert werden, dadurch gekennzeichnet, dass ein Proteinhydrolysat bei einem pH-Wert von 0,5 bis 1,0 mit Kochsalz gesättigt, mit dem genannten Alkohol Leucin-, Isoleucin- und Phenylalaninhydrochlorid neben geringem Anteil an Tyrosin- und Valinhydrochlorid extrahiert, durch Abdestillation des Alkohols eine Lösung der genannten Aminosäurehydrochloride erhalten, die Lösung über einen schwach basischen und über einen stark basischen Anionenaustauscher geleitet, der mit Phenylalanin und Tyrosin beladene stark basische Anionenaustauscher mit verdünnter Salzsäure eluiert, das phenylalanin- und tyrosinhaltige Eluat konzentriert, dessen Salzsäuregehalt auf 20-25% eingestellt, und nach Abtrennung von Natriumchlorid bei 70 bis 90°C Phenylalaninhydrochlorid durch Kühlung auskristallisiert und durch Umkristallisation aus 20 bis 25%iger Salzsäure weiter gereinigt wird.

2. Verfahren nach Anspruch 1, dadurch gekennzeichnet, dass das reine Phenylalaninhydrochlorid in Wasser gelöst, mit einem schwach basischen Anionenaustauscher die Salzsäure abgetrennt und durch Konzentrieren der neutralen Lösung reines L-Phenylalanin gewonnen wird.

3. Verfahren nach Anspruch 1, dadurch gekennzeichnet, dass durch Waschen des Alkoholextraktes mit Wasser dessen Gehalt an Tyrosin und Valin vermindert wird.

**Claims**

1. A process for recovering L-phenyl-alanine from protein hydrolysates, in which amino acid hydrochlorides are extracted from aqueous solutions with a water-immiscible alcohol, especially butanol or isobutanol, characterised by saturating a protein hydrolysate at a pH-value of 0.5 to 1.0 with sodium chloride, extracting hydrochlorides of leucine, isoleucine and phenyl-alanine besides a small part of hydrochlorides of tyrosine and valine with said alcohol, obtaining a solution of said amino acid hydrochlorides by distilling off the alcohol, passing the solution over a weakly basic and over a strongly basic anion exchanger, eluting the strongly basic anion exchanger charged with phenyl-alanine and tyrosine with diluted hydrochloric, concentrating the eluate containing phenyl-alanine and tyrosine, adjusting its hydrochloric acid content to between 20 and 25%, separating off sodium chloride at 70 to 90°C, crystallising out phenyl-alanine hydrochloride by cooling and further purifying it by recristallisation from 20 to 25% hydrochloric acid.

2. A process according to claim 1, characterized by diluting the pure phenyl-alanine hydrochloride in water, separating the hydrochloric acid with a weakly basic anion exchanger and concentrating the neutral solution for optaining pure L-phenyl-alanine.

3. A process according to claim 1, characterized by washing the alcohol extract with water for reducing its tyrosine and valine content.

**Revendications**

1. Procédé d'obtention de L-phénylalanine à partir d'hydrolysasts de protéines, dans lequel on extrait des chlorhydrates d'acides aminés d'une solution à l'aide d'un alcool non miscible à l'eau, notamment le butanol ou l'isobutanol, caractérisé par le fait que l'on sature en chlorure de sodium un hydrolysat de protéines à un pH de 0,5 à 1,0, on extrait avec ledit alcool des chlorhydrates de leucine, isoleucine et phénylalanine outre une petite partie de chlorhydrates de tyrosine et valine, on obtient une solution desdits chlorhydrates d'acides aminés par distillation de l'alcool, on fait passer cette solution sur un échangeur d'anions faiblement basique puis sur un échangeur d'anions fortement basique, on élue à l'acide chlorhydrique dilué l'échangeur d'anions fortement basique chargé de phénylalanine et de tyrosine, on concentre l'éluat contenant la phénylalanine at la tyrosine, on ajuste sa concentrationen en acide chlorhydrique à 20-25%, on sépare le chlorure de sodium à 70-90°C, on cristallise le chlorhydrate de phénylalanine par refroidissement et on le purifie par recristallisation de l'acide chlorhydrique à 20-25%.

2. Procédé selon la revendication 1, caractérisé par le fait que l'on dissout le chlorhydrate de phénylalanine pur dans l'eau, on sépare l'acide chlorhydrique à l'aide d'un échangeur d'anions faiblement basique et l'on concentre la solution neutre pour obtenir la L-phénylalanine pure.

3. Procédé selon la revendication 1, caractérisé par le fait que l'on diminue la teneur en tyrosine et valine de l'extrait alcoolique par lavage à l'eau.